# EUROPEAN PATENT SPECIFICATION

(11) **EP 2 544 563 B1**
(45) Date of publication and mention of the grant of the patent: **16.09.2015**
(21) Application number: 11710548.6
(22) Date of filing: 08.03.2011
(51) Int. Cl.: A61B 17/322, A45D 34/04, A45D 29/00, A61B 17/54

(54) **SKIN TREATING DEVICE**
HAUTBEHANDLUNGSVORRICHTUNG
DISPOSITIF DE TRAITEMENT DE LA PEAU

(30) Priority: 12.03.2010 GB 201004172
(43) Date of publication of application: 16.01.2013
(73) Proprietor: LRC Products Limited, Slough, Berkshire SL1 3UH (GB)
(72) Inventor: COULTER, Francis Joseph, Chorley PR6 9ST (GB); WARD, Jonathan, Hull HU8 7DS (GB)
(74) Representative: O'Brien, Niall James
(86) International application number: PCT/GB2011/050451
(87) International publication number: WO 2011/110840

(56) References cited:
- WO-A1-03/024290
- WO-A1-2006/068638
- FR-A1- 2 896 678
- FR-A1- 2 928 533
- US-A- 3 574 251
- US-A1- 2007 240 730
- US-A1- 2008 242 204
- US-A1- 2009 254 055

## Description

This invention relates to a hand-held device for treating hard skin. The device is especially adapted for removing hard skin, particularly unsightly hard skin such as calluses, from the feet or hands.

Calluses are areas of thickened skin that may occur on the hands or feet and can be caused by persistent rubbing or uneven pressure, for example from ill-fitting shoes. They have a tendency to form over bony prominences and with regards the feet, they are most commonly found at the heel, the ball of the foot and the sides of the toes. On the hand they typically form on the underside of the fingers or on the palm. Calluses are often unsightly, and the thicker they are the more yellow they can look. With time, particularly thick calluses can become cracked and painful.

There are a number of known methods for reducing or removing calluses, generally based upon rubbing, scraping or cutting the hardened skin away. In many cases this may include a first step of softening the skin by soaking the feet or hands in water or by applying some form of softening lotion to the hard skin. The hard skin can be reduced or removed using a device with an abrasive surface, such as a pumice stone, emery board or a device with carborundum paper attached to it. These devices can be manual or electrically operated and in the latter case, an abrasive head can be vibrated and rotated over the hard skin. Examples of such devices are described in WO03/022175.

The known abrasive devices have a number of drawbacks. In many cases they are unable to conform to the curvature of the parts of the hands or feet where calluses usually occur. In addition, abrasive surfaces tend to wear out with time and they can be difficult to clean, potentially leading to hygiene issues.

The majority of the prior art devices which remove skin with a cutting action incorporate one or more flat metal blades, such as a razor blade or the like. US 2005/0061343 discloses an example of such a cutting device in which a cutting blade is mounted in a head piece attached to a handle in order to be placed in contact with the skin and pulled across the callus for removing a thin layer of skin, the process being repeated as required. Such devices which shave off layers of skin are difficult for untrained people to use safely and often do not discriminate well between callused and healthy skin, so that they can present a significant risk of injury, e.g. due to cutting as a result of the blade being positioned incorrectly or being manipulated so that too much skin is removed.

Some known cutting devices are equipped with one or more curved blades instead of flat blades, the idea being that curved blades will conform more closely to the shaped surface of the foot or hand. An example of such device is described in US2007/0244491. There are also known callus removers with blades in the form of rasp foils which have a resemblance to cheese graters. These devices are subject to the same drawbacks as outlined above for devices with flat blades.

In US 2008/0230081 there is described a device for removing hard layers of skin having a body in the form of a disc with several concentric blade edges integrally formed on one side. One embodiment includes a number of such discs disposed in a rectangular array on one side of a planar member having a handle attached at one end, this device being made by injection moulding. The effectiveness of the individual blade edges is impaired due to the proximity and large number of blade edge portions that are configured to contact the skin at the same time.

Another approach to a callus-cutting device is described in US2007/0240730 and consists of a single stainless steel tube, one end of which is sharpened at its inner edge to provide a circular cutting edge. This callus remover is used by holding the tube perpendicular to the skin and moving the cutting edge back and forth with a reciprocating action for scraping off hard skin. The device is small and difficult to handle. Also, although the scraping effect is optimum when the tube is held perpendicular to the skin, users tend to hold it at an angle, which can make it ineffective or lead to excessive cutting depth. US 2007/0240730 also discloses that the steel tube may be filled with a wetting substance or skin softener which can be discharged through the centre of the cutting edge by means of a plunger inserted into the tube. Such an arrangement for dispensing a fluid is awkward to use and inconvenient due to delivery of the fluid at the cutting edge so that great care is needed for applying the liquid to a skin area before treating the skin with the cutting edge.

The present invention aims to provide a device which is more convenient and easier to use than the foregoing prior art devices for removing hard skin. In accordance with this objective the present invention provides a hand-held device for treating hard skin comprising a barrel for holding the device in use, a fluid reservoir within the barrel, a dispensing device mounted at a first end of the barrel for delivery of fluid from the reservoir for application to the skin to be treated, and a cutting head mounted at a second end of the barrel for acting on the skin, the cutting head comprising at least one cutting element having a substantially annular cutting edge for contact with the skin surface to be treated.

The barrel forms a convenient-handle for holding the device both when applying the liquid to the skin by means of the dispenser and when acting on the skin with the cutting head. Also, when the liquid is being applied the cutting head is held well away from the skin surface for enhanced safety.

In a preferred embodiment the cutting head comprises a base, and at least two cutting elements projecting from the base in spaced relationship to each other. Each cutting element can define a substantially annular cutting edge for contact with a skin surface to be treated, and each cutting edge can lie in a plane which is inclined to a skin contact plane defined by the extremities of the cutting elements. Such a device provides effective hard skin removal and is also easy and safe to use, even for unskilled users. Providing the dispenser for fluid and cutting head integrated into a common hand-held device also ensures greater convenience to the user. The device may comprise two or more cutting elements that are conveniently formed by stainless steel tubes, with each tube having a cutting edge at the skin-contacting end. When the cutting edges are inclined to the skin contact plane there can be a small contact area between the cutting edges and the skin so a small force can be used to press the cutting elements against the skin. Also, with there being at least two, and preferably more cutting elements the force with which the device is pressed against the skin by a user is spread over a number of contact points or areas so that the risk of an individual cutting edge being applied with excessive force and possibly cutting too deeply into the skin is substantially reduced. This advantage is secured even when the cutting elements are inclined as described below. Thus the device of the invention is safe for use by unskilled as well as experienced users because there is less risk of inadvertent injury due to an individual cutting element being applied with sufficient force to an excessive cutting depth.

Each cutting edge lies in a plane inclined to a skin contact plane that contains the extremities of the cutting elements and can be substantially parallel to the base. The direction of inclination is such that the leading portion of the annular cutting edge is at the greatest distance from the skin contact plane. The inclination of the planes of the cutting edges to the skin contact plane can be from 5° to 45°, such as from 10° to 25°, and in the particular embodiment specifically described herein below is about 15 °.

The cutting elements are conveniently tubular, and more especially right circular cylindrical tubes with the cutting edge of each tube lying in a plane perpendicular to the tube axis. The cutting elements can be arranged to project from the base along parallel axes. Although other constructions can easily be envisaged, for convenience of manufacture the base is preferably moulded from rigid plastics material, and provided with sockets to receive the respective cutting elements which can be fixed to the base by being pushed into the sockets with a tight friction fit.

The exact number of cutting elements is not critical, but in the interest of efficiency it is preferable for there to be at least three, and possibly up to five or more, and for the cutting elements to be disposed in a staggered array so that skin missed by the leading cutting elements passing over the skin first may be acted upon by another cutting element that follows. One suitable arrangement is for three cutting elements to be positioned at the corners of a triangle with the apex of the triangle being directed either forwardly or backwardly. Another possibility would be to have four cutting elements positioned at the corners of a diamond shape. Especially effective is an arrangement in which the cutting elements are positioned in at least two rows extending transversely to the movement direction, the cutting elements in one row being staggered with respect to the cutting elements of another row. For example, five cutting elements could be arranged with three in a first row, and two in a second row so as to cover the gaps between the cutting elements in the first row. The rows of cutting elements may extend along straight, V-shaped or curved lines. An effective arrangement of cutting elements has been found to be one in which the numbers of cutting elements in successive rows reduces from front to rear along the movement direction.

For ease of skin debris clearance it is preferred that the interiors of the tubular cutting elements are connected via openings in the base to a cavity in the device, which cavity is preferably open to the exterior of the device, although it could be closed by a cover that can be opened for rinsing and cleaning purposes. The cavity can be suitably formed by an open-topped recess in the upper side of the base.

The barrel has a size and shape to facilitate gripping in the hand. The barrel axis may extend at a shallow angle to the skin contact plane of the cutting elements, which facilitates movement of the cutting elements across a hard skin area by pulling on the barrel. The barrel may include an inner barrel enclosing the reservoir and an outer barrel, which can facilitate assembly of the device, with the dispensing device and inner barrel pre-assembled as a unit for insertion into the outer barrel. Thus, the dispensing device may be fitted to the inner barrel. The cutting head may have mounting parts respectively engaging with the inner and outer barrels.

The fluid dispensing device preferably comprises an applicator for applying the skin treatment fluid to the skin, such as a roller ball applicator, which may be especially efficacious if the fluid comprises a gel, such as a skin softening fluid comprising a hydrating gel.

Other forms of dispenser and applicator are of course possible. For example in a manner known per se the dispenser may comprise a valve to control delivery of fluid from the reservoir, and possibly opened either by squeezing the reservoir or by pressing the applicator against the skin, and the applicator may comprise a flexible element such as a foam pad or sponge for spreading the fluid over the skin surface.

Rather than a fluid for softening hard skin prior to removal by treatment with the cutting head, the reservoir may comprise a fluid such as a soothing liquid or gel intended to be applied to the skin after treatment with the cutting head to remove hard skin.

The above and other features and advantages of the invention will become apparent from the following detailed description of a preferred embodiment, which is given by way of example and with reference to the accompanying drawings in which:-
- Figure 1: is a perspective view of a hand-held device according to the invention and shown with the end caps removed;
- Figure 2: is a side elevation of the device shown in Figure 1;
- Figure 3: is an underneath plan view of the device shown in Figure 1;
- Figure 4: is a bottom view of the base plate of the cutting head of the device shown in Figure 1;
- Figure 5: is a top view of the base plate of Figure 4;
- Figure 6: is a longitudinal cross-section through the base plate taken along the line A-A in Figure 4;
- Figure 7: is an enlarged scale axial cross-section through one of the cutting elements fitted into the device shown in Figure 1;
- Figure 8: is a side elevation showing the device of Figure 1 with the end caps fitted;
- Figure 9: is an axial cross-section through the device taken along the line B-B in Figure 8;
- Figure 10: is an axial cross-section through the roller ball sub-assembly;
- Figure 11: is an enlarged axial section through the roller ball seat member;
- Figure 12: is an axial cross-section through another hand held device according to the invention; and
- Figure 13: is an enlarged scale view of the cutting head of the device shown in Figure 12.

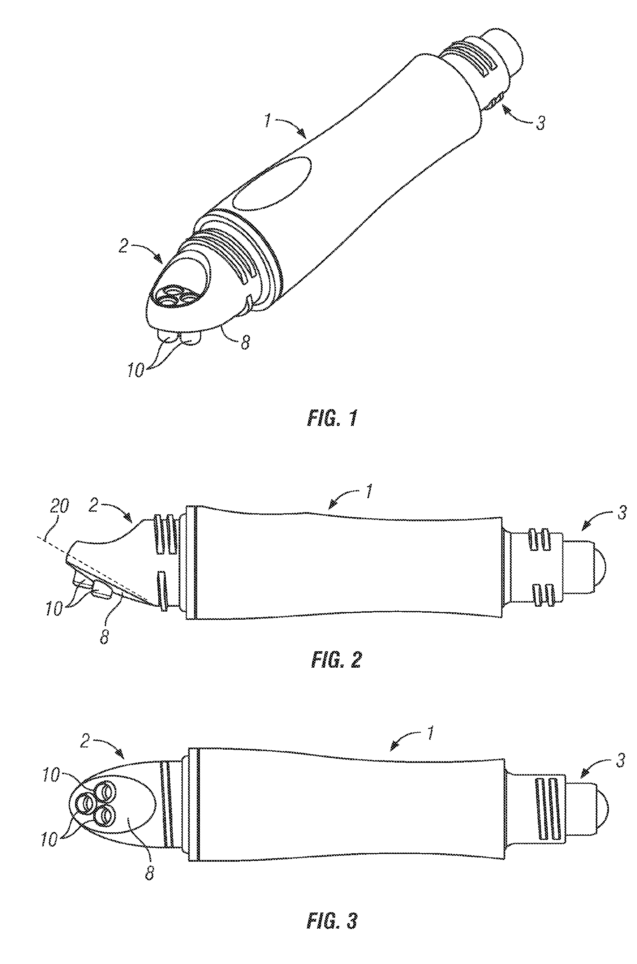

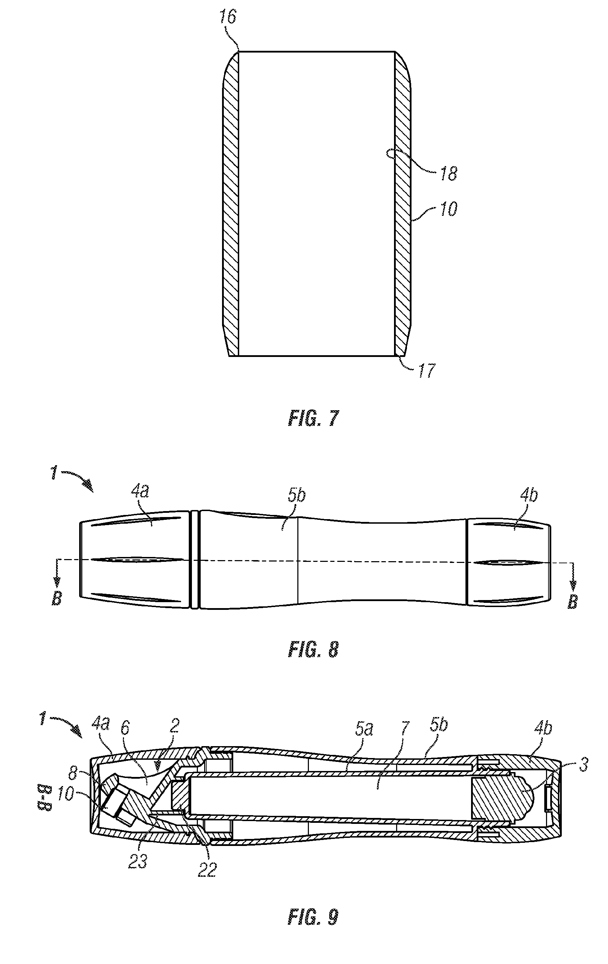

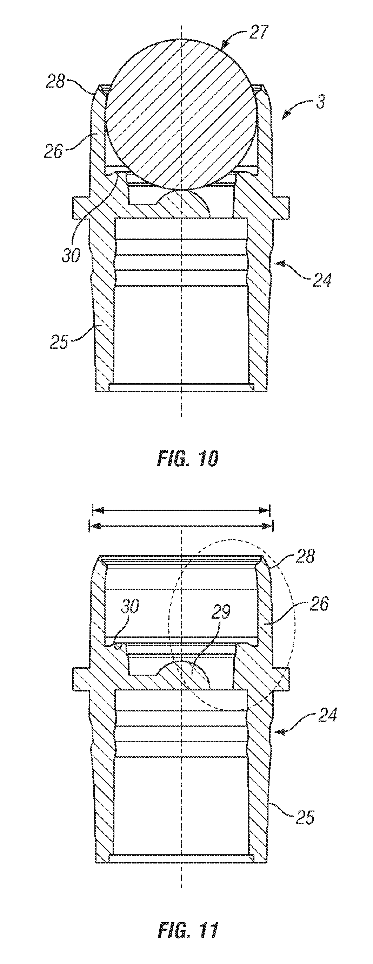

Illustrated in Figures 1 to 11 of the drawings is a device in the form of a hand tool for softening and removing hard skin, such as calluses on the feet or hands of a user of the device. The device comprises an elongate generally cylindrical barrel 1 which is provided a working or cutting head 2 at one end and a fluid dispenser and applicator 3 at the other end. Removable end caps 4a and 4b are provided for enclosing the cutting head 2 and the fluid dispenser/applicator 3 respectively when not in use. The barrel 1 includes an inner barrel 5a which defines a reservoir 7 for holding and storing a skin treat fluid as described further below, and an outer barrel 5b which surrounds the inner barrel coaxially. As may be seen in Figure 9, the inner and outer barrels are interconnected adjacent one end and may be conveniently moulded in one piece.

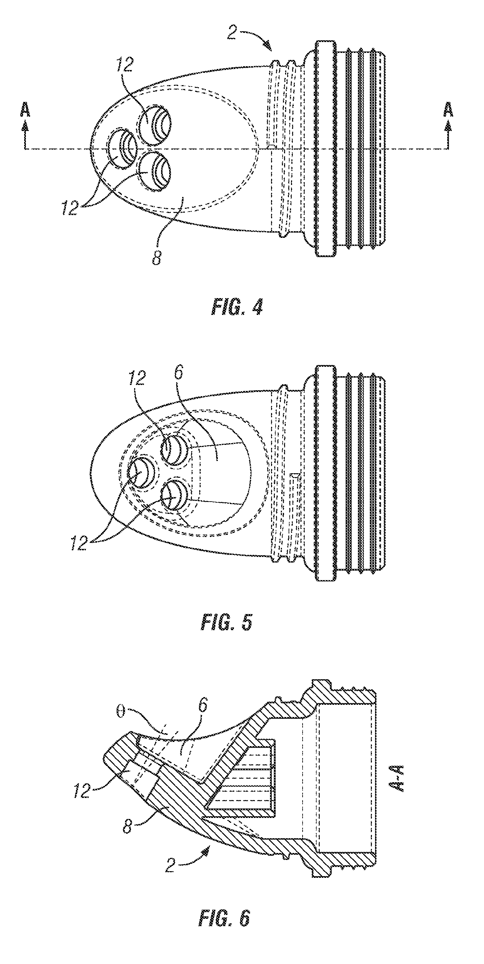

The cutting head 2 has a tubular mounting part that is firmly fixed into the free end of the outer barrel 5b, and a working part comprising a nose portion or base 8. Several cutting elements 10 in the form of stainless steel tubes are fixedly fitted to the base 8. As shown in Figures 4-6, the bottom of base 8 is substantially flat and is formed with an array of sockets 12 adapted to receive the respective tubular cutting elements 10, the inner ends of the sockets being open at the upper side of the base into a shallow cavity 6 formed on the upper side of the base. The sockets 12 are cylindrical and have their axes parallel and inclined at an angle θ of about 15° to a perpendicular to the plane of the bottom of the base. The sockets 12 are disposed for mounting the cutting elements 10 in a predetermined array further described below.

Each cutting element 10 consists of a right circular cylindrical stainless steel tube as shown in cross-section in Figure 7. At the outer end each tube is tapered to define a circular cutting edge 16 at the end of the inner surface 18 of the tube. The inner end of the tube is chamfered to assist insertion into a socket 12 of the base 8, but has a blunt end face 17. The tubes 10 are dimensioned with an outer diameter slightly greater than the diameter of the sockets 12 and the tubes 10 are pushed into the sockets so that they are retained securely by the resulting tight friction fit. With cutting tubes fully inserted into the eight sockets 12 of the base, the tubes are set coaxially with the sockets and as a result the cutting edge 16 of each tube 10 lies in an inclined plane that is at an angle of 15 ° to the contact plane 20 defined by the extremities of the cutting tubes 10.

The pattern of the sockets 12 in the base is such that the cutting tubes 10 are disposed in a triangular array. In effect there are two cutting elements in a first row and one cutting element in a second row and staggered relative to those of the first row so as to span the gap between the cutting tubes of the first row. In this way it can be ensured that there will be no skin area that is located between other skin areas acted on by cutting elements and that will not be itself acted on by a cutting element when the device is applied to and moved across the skin. Other numbers of cutting elements and arrangements on the base of the cutting head can readily be envisaged. Indeed many different arrays for the cutter tubes on the base are possible.

The hollow interiors of the tubular cutting elements 10 are connected to the cavity 6 in the device so that particles of hard skin that are removed by the cutting and scraping action of the cutting edges and which may collect in the tubes, and/or in the cavity can be easily emptied out of the device, and such cleaning can be aided further by rinsing with water.

The inclination of the cutting elements, means that the plane of the cutting edge of each element will be inclined at an angle from 5 ° to 45 °, specifically 15 ° in the illustrated embodiment, to the surface of a skin callus being treated with the device, which will ensure very effective skin removal when the device is pulled by means of the barrel 1 across the callus with the cutting elements in contact with the skin surface. An appropriate diameter for the circular cutting edges is from 3 to 5mm, such as around 4mm.

The end of the inner barrel 5a adjacent the cutting head 2 is closed by an end wall which has an axially directed projection 22 that engages with a close fit into a socket 23 formed on the cutting head, this engagement between the inner barrel and the cutting head providing additional strength and rigidity to the structure of the device. At its opposite open end the inner barrel 5a is fitted with the dispenser 3 for dispensing a skin treatment fluid contained in the reservoir 7 enclosed by the inner barrel. The dispenser preferably comprises an applicator that is intended to be used to apply the fluid to the skin surface and to spread the fluid over the area of the skin to be treated. In the illustrated embodiment the dispenser comprises a roller ball applicator of conventional construction, this applicator comprising a seat member 24 with a cylindrical mounting portion 25 that is inserted with a tight fit into the open end of the inner barrel 5a and a socket portion 26 in which the ball 27 is received and is held captive by an inturned lip at the free edge 28 of the socket. The fluid in the reservoir 7 is free to contact the inwardly facing portion of the ball surface through a central opening extending through the seat member 25 between the mounting and socket portions. An integral spring element 29 is provided to act on the ball to urge it away from a sealing rim 30 formed at the innermost end of the socket so that the fluid can be transferred and applied to the skin surface by applying the ball 27 to the skin surface and rolling it over the surface in a manner well known per se. When the cap 4b is fitted over the dispenser 3 it may press the ball 27 against the sealing rim 30 to help prevent leakage of fluid from the reservoir 7, during periods of non-use. The removable caps 4a, 4b have internal screw threads for cooperation with external screw threads provided on the cutting head 2 and the end portion of the inner barrel receiving the dispenser 3, respectively. The fluid to be dispensed and applied to the skin to be treated is preferably a skin softening fluid, in particular a hydrating gel. By means of the device of the invention the hydrating gel can easily and conveniently be applied to the skin while the cutting head 2 is held at a safe distance clear of the skin surface and ideally covered by the removable cap 4a. After the hard skin to be removed has been suitably softened, the cutting head 2 can be used to scrape the hard skin away by drawing the cutting elements 10 over the skin surface as described above. In addition to being effective in applying the fluid to the skin surface the roller ball applicator can have a beneficial effect by massaging the skin being treated.

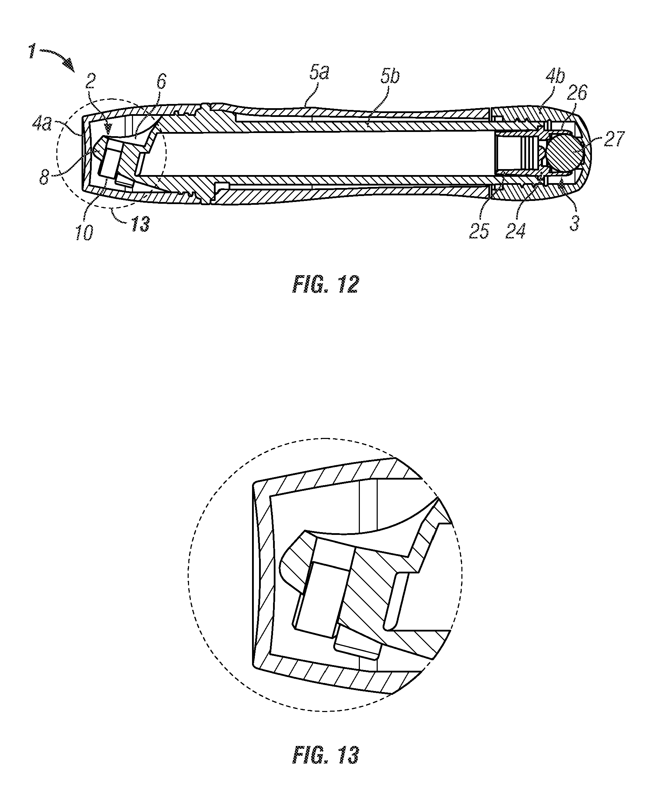

The hand-held device shown in Figures 12 and 13 is for the most part the same as that described above with reference to Figures 1 to 11 and only the differences will be described. In the embodiment of Figures 12 and 13, the inner barrel 5b is integral with the cutting head 2, and the outer barrel 5a is a separate sleeve fitted over the inner barrel and having circumferential grooves at its opposite ends for interlocking with complementary circumferential ribs formed on the inner barrel.

Other modifications are of course possible within the scope of the invention as defined by the following claims and will occur to those skilled in the art. As examples there are mentioned the possibility to use applicators comprising multiple roller balls, a sponge or a brush, and the possibility to provide instead of a skin softening agent a fluid for application to the skin after treatment with the cutting head to remove hard skin, such as for soothing or friction reducing purposes. Also, rather than a gel, a liquid, lotion or cream can be applied.

## Claims

1. A hand-held device for treating hard skin comprising a barrel (1) for holding the device in use, a fluid reservoir (7) within the barrel (1), a dispensing device mounted at a first end of the barrel (1) for delivery of fluid from the reservoir (7) for application to the skin to be treated,
**characterised in that** there is a cutting head (2) mounted at a second end of the barrel (1) for acting on the skin, the cutting head (2) comprising at least one cutting element (10) having a substantially annular cutting edge for contact with the skin surface to be treated.

2. A hand-held device according to claim 1, wherein the cutting head (2) comprises a base (8) and at least two cutting elements (10) projecting from the base (8) and spaced apart from each other.

3. A hand-held device according to claim 2, wherein the cutting elements (10) have extremities lying in a skin contact plane (20) and each cutting edge lies in a plane inclined to the skin contact plane (20).

4. A hand-held device according to claim 3, wherein the inclined plane is at an angle in the range of from 5⁰ to 45⁰, preferably from 10⁰ to 25⁰, to the skin contact plane (20).

5. A hand-held device according to any one of the claims 2 to 4, wherein the cutting elements (10) are tubular.

6. A hand-held device according to claim 5, wherein the cutting elements (10) are right circular cylindrical tubes, and the cutting edge of each cutting elements (10) lies in a plane perpendicular to the axis of the cutting element (10).

7. A hand-held device according to any one of claims 2 to 6, wherein the cutting elements (10) project from the base (8) along parallel axes.

8. A hand-held device according to any one of the preceding claims, wherein there are three or more cutting elements (10) positioned in a staggered array.

9. A hand-held device according to claim 8, wherein the cutting elements (10) are arranged in at least two rows extending transversely to a movement direction, the cutting element(s) (10) in one row being staggered with respect to the cutting elements (10) of another row.

10. A hand-held device according to any one of claims 2 to 9, wherein the base (8) comprises a plate to which the cutting elements (10) are fixed.

11. A hand-held device according to claim 10, wherein the plate has sockets (12) into which the respective cutting elements (10) are inserted.

12. A hand-held device according to claim 11, wherein the sockets (12) have inner ends that are open to a cavity (6) formed in the cutting head (2).

13. A hand-held device according to claim 12, wherein the cavity (6) is open to the exterior.

14. A hand-held device according to any one of the preceding claims, wherein the barrel (1) includes an inner barrel (5a) enclosing the reservoir and an outer barrel (5b) surrounding the inner barrel (5a).

15. A hand-held device according to claim 14, wherein the cutting head (2) has mounting parts respectively engaged with the inner and outer barrels (5a,5b)

16. A hand-held device according to claim 14 or 15, wherein the dispensing device is fitted into the inner barrel (5a).

17. A hand-held device according to any one of the preceding claims wherein the dispensing device comprises an applicator (27) for applying the fluid to the skin.

18. A hand-held device according to claim 17, wherein the applicator (27) is a roller ball applicator.

19. A hand-held device according to any one of the preceding claims wherein the reservoir (7) contains a fluid for softening hard skin.

20. A hand-held device according to any of the preceding claims wherein the reservoir (7) contains a hydrating gel.

## Patentansprüche

1. Handgerät zur Behandlung von Hornhaut, umfassend einen Zylinder (1) zum Halten des Geräts im Gebrauch, ein Flüssigkeitsreservoir (7) in dem Zylinder (1), eine Abgabevorrichtung, die an einem ersten Ende des Zylinders (1) befestigt ist, zur Abgabe von Flüssigkeit aus dem Reservoir (7) zur Applikation auf die zu behandelnde Haut, **dadurch gekennzeichnet, dass** ein Schneidkopf (2) an einem zweiten Ende des Zylinders (1) zum Einwirken auf die Haut befestigt ist, wobei der Schneidkopf (2) zumindest ein Schneidelement (10) umfasst, das eine im Wesentlichen ringförmige Schneidkante zur Berührung der zu behandelnden Hautoberfläche aufweist.

2. Handgerät nach Anspruch 1, worin der Schneidkopf (2) eine Basis (8) und zumindest zwei von der Basis (8) vorstehende und voneinander im Abstand angeordnete Schneidelemente (10) umfasst.

3. Handgerät nach Anspruch 2, worin die Schneidelemente (10) Extremitäten aufweisen, die in einer Hautkontaktebene (20) liegen und jede Schneidkante in einer Ebene liegt, die zur Hautkontaktebene (20) geneigt ist.

4. Handgerät nach Anspruch 3, worin die geneigte Ebene in einem Winkel im Bereich von 5° bis 45°, vorzugsweise von 10° bis 25°, zur Hautkontaktebene (20) liegt.

5. Handgerät nach einem der Ansprüche 2 bis 4, worin die Schneidelemente (10) röhrenförmig sind.

6. Handgerät nach Anspruch 5, worin die Schneidelemente (10) gerade kreiszylinderförmige Röhren sind und die Schneidkante jedes Schneidelements (10) in einer Ebene liegt, die senkrecht zu der Achse des Schneidelements (10) ist.

7. Handgerät nach einem der Ansprüche 2 bis 6, worin die Schneidelemente (10) von der Basis (8) entlang parallelen Achsen vorstehen.

8. Handgerät nach einem der vorhergehenden Ansprüche, worin drei oder mehr Schneidelemente (10) in einer versetzten Reihe angeordnet sind.

9. Handgerät nach Anspruch 8, worin die Schneidelemente (10) in mindestens zwei Reihen angeordnet sind, die sich quer zu einer Bewegungsrichtung erstrecken, wobei das bzw. die Schneidelement(e) (10) in einer Reihe bezüglich der Schneidelemente (10) einer anderen Reihe versetzt ist bzw. sind.

10. Handgerät nach einem der Ansprüche 2 bis 9, worin die Basis (8) eine Platte umfasst, an der die Schneidelemente (10) befestigt sind.

11. Handgerät nach Anspruch 10, worin die Platte Aufnahmebuchsen (12) aufweist, in welche die jeweiligen Schneidelemente (10) eingeführt werden.

12. Handgerät nach Anspruch 11, worin die Aufnahmebuchsen (12) Innenenden aufweisen, die zu einem in dem Schneidkopf (2) geformten Hohlraum (6) hin offen sind.

13. Handgerät nach Anspruch 12, worin der Hohlraum (6) zur Außenseite offen ist.

14. Handgerät nach einem der vorhergehenden Ansprüche, worin der Zylinder (1) einen Innenzylinder (5a), der das Reservoir umschließt, und einen Außenzylinder (5b), der den Innenzylinder (5a) umgibt, aufweist.

15. Handgerät nach Anspruch 14, worin der Schneidkopf (2) Befestigungsteile aufweist, die jeweils mit den Innen- und Außenzylindern (5a, 5b) in Eingriff stehen.

16. Handgerät nach Anspruch 14 oder 15, worin die Abgabevorrichtung in dem Innenzylinder (5a) angebracht ist.

17. Handgerät nach einem der vorhergehenden Ansprüche, worin die Abgabevorrichtung einen Applikator (27) zur Applikation von Flüssigkeit auf die Haut umfasst.

18. Handgerät nach Anspruch 17, worin der Applikator (27) ein Rollapplikator ist.

19. Handgerät nach einem der vorhergehenden Ansprüche, worin das Reservoir (7) eine Flüssigkeit zum Aufweichen von Hornhaut enthält.

20. Handgerät nach einem der vorhergehenden Ansprüche, worin das Reservoir (7) ein hydratisierendes Gel enthält.

## Revendications

1. Dispositif tenu à la main destiné au traitement de la couche cornée de la peau comportant un cylindre (1) destiné à tenir le dispositif en cours d'utilisation, un réservoir (7) de fluide à l'intérieur du cylindre (1),
un dispositif de distribution monté au niveau d'une première extrémité du cylindre (1) destiné à administrer le fluide depuis le réservoir (7) pour appliquer ce dernier sur la peau devant être traitée,
**caractérisé en ce que**, une tête de coupe (2) est montée au niveau d'une seconde extrémité du cylindre (1) destinée à agir sur la peau, la tête (2) de coupe comportant au moins un élément (10) de coupe présentant un bord de coupe sensiblement annulaire destiné à venir en contact avec la surface de la peau à traiter.

2. Dispositif tenu à la main selon la revendication 1, la tête (2) de coupe comportant une base (8) et au moins deux éléments (10) de coupe faisant saillie depuis la base (8) et espacés l'un de l'autre.

3. Dispositif tenu à la main selon la revendication 2, les éléments (10) de coupe présentant des extrémités reposant dans un plan (20) de contact avec la peau et chaque bord de coupe reposant dans un plan incliné par rapport au plan (20) de contact avec la peau.

4. Dispositif tenu à la main selon la revendication 3, le plan incliné formant un angle compris entre 5 et 45°, de préférence entre 10 et 25°, par rapport au plan (20) de contact avec la peau.

5. Dispositif tenu à main selon l'une quelconque des revendications 2 à 4, les éléments (10) de coupe étant tubulaires.

6. Dispositif tenu à la main selon la revendication 5, les éléments (10) de coupe étant des tubes cylindriques circulaires droits, et le bord de coupe de chaque élément (10) de coupe reposant dans un plan perpendiculaire à l'axe de l'élément (10) de coupe.

7. Dispositif tenu à la main selon l'une quelconque des revendications 2 à 6, les éléments (10) de coupe faisant saillie depuis la base (8) le long d'axes parallèles.

8. Dispositif tenu à la main selon l'une quelconque des revendications précédentes, les éléments (10) de coupe étant au nombre de trois ou plus positionnés en un réseau en quinconce.

9. Dispositif tenu à la main selon la revendication 8, lesdits éléments (10) de coupe étant agencés sur au moins deux rangées qui s'étendent transversalement à une direction de mouvement, le ou les éléments (10) de coupe sur une rangée étant décalés par rapport aux éléments (10) d'une autre rangée.

10. Dispositif tenu à la main selon l'une quelconque des revendications 2 à 9, la base (8) comportant une plaque à laquelle les éléments (10) de coupe sont fixés.

11. Dispositif tenu à la main selon la revendication 10, la plaque présentant des emboîtures (12) dans lesquelles les éléments (10) de coupe respectifs sont insérés.

12. Dispositif tenu à la main selon la revendication 11, les emboîtures (12) ayant des extrémités internes qui sont ouvertes sur une cavité (6) formée dans la tête (2) de coupe.

13. Dispositif tenu à la main selon la revendication 12, la cavité (6) étant ouverte sur l'extérieur.

14. Dispositif tenu à la main selon l'une quelconque des revendications précédentes, le cylindre (1) comprenant un cylindre (5a) interne enfermant le réservoir et un cylindre (5b) externe entourant le cylindre (5a) interne.

15. Dispositif tenu à la main selon la revendication 14, la tête (2) de coupe ayant des parties de montage qui sont respectivement mises en prise avec les cylindres (5a, 5b) interne et externe.

16. Dispositif tenu à la main selon la revendication 14 ou 15, le dispositif de distribution étant installé dans le cylindre (5a) interne.

17. Dispositif tenu à la main selon l'une quelconque des revendications précédentes, le dispositif de distribution comportant un applicateur (27) destiné à appliquer le fluide sur la peau.

18. Dispositif tenu à la main selon la revendication 17, l'applicateur (27) étant un applicateur à bille roulante.

19. Dispositif tenu à la main selon l'une quelconque des revendications précédentes, le réservoir (7) contenant un fluide destiné à ramollir la couche cornée de la peau.

20. Dispositif tenu à la main selon l'une quelconque des revendications précédentes, le réservoir (7) contenant un gel hydratant.
